# EUROPEAN PATENT SPECIFICATION

(11) **EP 3 210 211 B1**
(45) Date of publication and mention of the grant of the patent: **17.02.2021**
(21) Application number: 15793889.5
(22) Date of filing: 22.10.2015
(51) Int. Cl.: G21G 1/00, B01D 57/02

(54) **RADIOISOTOPE RECOVERY**
RADIOISOTOPRÜCKGEWINNUNG
RÉCUPÉRATION DE RADIO-ISOTOPES

(30) Priority: 23.10.2014 GB 201418895
(43) Date of publication of application: 30.08.2017
(73) Proprietor: The University Of Hull, Hull, Humberside HU6 7RX (GB)
(72) Inventor: ARCHIBALD, Stephen James, Hull Humberside HU6 7RX (GB); HE, Ping, Hull Humberside HU6 7RX (GB); HASWELL, Stephen John, Hull Humberside HU6 7RX (GB); PAMME, Nicole, Hull Humberside HU6 7RX (GB); BROWN, Nathan Joel, Hull Humberside HU6 7RX (GB); TARN, Mark Duncan, Hull Humberside HU6 7RX (GB); ALEXANDER, Richard, Hull Humberside HU6 7RX (GB)
(74) Representative: Secerna LLP
(86) International application number: PCT/GB2015/053170
(87) International publication number: WO 2016/063069

(56) References cited:
- US-A1- 2009 095 635
- US-A1- 2010 069 600
- US-A1- 2010 101 943
- US-A1- 2011 100 840

## Description

The present invention relates to a method and an apparatus for separating and recovering a radioisotope from a solution. More particularly, certain embodiments of the invention relate to a method for recovering a radioisotope from a solution by electro-trapping and release using a microfluidic cell. The radioisotope may subsequently be used in the preparation of radiopharmaceuticals.

### Background to the invention

Positron emission tomography (PET) is a powerful molecular imaging technique that allows the study and visualisation of the molecular and cellular processes of human physiology which can provide important information about metabolism and diseases such as cancer. The most common positron-emitting radioisotopes suitable for incorporation into organic structures are fluorine-18 (¹⁸F), carbon -11 (¹¹C), nitrogen-13 (¹³N) and oxygen-15 (¹⁵O). Because of its appropriate half-life (109.8 min) allowing sufficient time for multistep synthetic labelling reactions and transportation of doses to sites several hours away, and a low positron energy giving high resolution images, ¹⁸F has become the most widely used and commonly available radioisotope for PET imaging. Currently, [¹⁸F]-2-Fluoro-2-deoxy-β-D-glucopyranose (also known as [¹⁸F]-Fluorodeoxyglucose or [¹⁸F]-FDG) is the most frequently used radiopharmaceutical for PET investigations.

Radiopharmaceuticals such as [¹⁸F]-FDG can be synthesized by nucleophilic substitution of a precursor compound. The process usually starts with recovery of ¹⁸F fluoride ions from ¹⁸O-enriched water into an appropriate solvent using an anion exchange separation cartridge, followed by azeotropic evaporation to remove any residual water. The ¹⁸F fluoride then reacts with a precursor through nucleophilic substitution.

Historically, PET tracers were produced in large batches at centralised cyclotron or decay generator facilities and then transported as multiple doses to the imaging sites, usually a hospital, to be administered to multiple patients during pre-arranged PET clinics. However, recent technological advances mean that individual imaging sites are able to have mini-PET cyclotrons on site thus allowing small volumes of radioisotopes such as ¹⁸F to be produced for the on-demand synthesis of a single dose of radiotracer i.e. "dose-on-demand".

Currently, the dose-on-demand approach is not achievable with traditional vessel-based techniques. In addition, current methods, based on either distillation and/or the combination of solvent-exchange on an anion exchange column and azeotropic evaporation step for the isolation of ¹⁸F from its aqueous environment, are time-consuming, require complex automation and reduce radiochemical yield. The extremely high surface-to-volume ratio and small size of microreactors combining with flow chemistry have significant potential to facilitate such chemistry in terms of higher yields, shorter reaction times, reduced consumption and lower environmental impact. Azeotropic evaporation is, however, very difficult to implement on such miniaturized systems. Alternative methods based on an electrochemical cell have been reported in which ¹⁸F is trapped and then directly released into organic solvent for the preparation of radiotracers without the need for an evaporation step. However, disadvantages of the known processes include low efficiency of trapping and/or release, reliance on high voltages or the use of complex electrochemical cells.

The present invention has been devised with these issues in mind.

Certain embodiments of the present invention aim to overcome the problems associated with the prior art.

### Summary of Certain Embodiments of the Present Invention

According to a first aspect of the invention there is provided a method for separating and recovering a radioisotope from an aqueous solution comprising the radioisotope, the method comprising using a microfluidic device comprising a chamber:
flowing the aqueous solution to the chamber, the chamber comprising a first electrode and a second electrode;
generating a first electric field between the first and second electrodes, thereby trapping the radioisotope on the first electrode;
flowing an organic-based solution to the chamber comprising the first and the second electrodes; and
generating a second electric field between the first and the second electrodes, wherein the second electric field has an opposing polarity to the first electric field, thereby releasing the radioactive isotope from the first electrode into the organic based solution, wherein the first electrode is formed from a carbon rod or section thereof and has a flat surface comprising a plurality of recesses.

Aptly, the chamber is comprised in a microfluidic chip. Aptly, the method comprises using a microfluidic chip. In an embodiment, the method comprises providing a microfluidic chip comprising the first electrode and the second electrode.

According to a further aspect of the invention, there is provided a microfluidic apparatus for separating and recovering a radioisotope from an aqueous solution comprising the radioisotope, the apparatus comprising:
an inlet;
an outlet; and
a chamber in fluid communication with the inlet and the outlet to form a fluid pathway, the chamber comprising a first electrode and a second electrode, wherein the first electrode is formed from a carbon rod and has a flat surface comprising a plurality of recesses; wherein the chamber has a volume capacity of no greater than about 50 µL; and
wherein the distance between the first electrode and the second electrode is no greater than 0.5 mm.

In certain embodiments, the microfluidic apparatus may be comprised on a microfluidic chip.

The radioisotope may be any desired radioisotope, such as a radioisotope for the preparation of a radiotracer. In some embodiments, the radioisotope is selected from ¹⁸F, ^{99m}Tc, ¹¹C and ⁶⁸Ga.

In some embodiments, the radioisotope is a radioactive fluoride isotope, also referred to herein as ¹⁸F-fluoride or ¹⁸F.

In some embodiments, the aqueous solution comprises ¹⁸O-enriched water. The ¹⁸O-enriched water may comprise ¹⁸F.

In some embodiments the method comprises providing the aqueous solution at a flow rate of no greater than approximately 1 mL/min. In some embodiments the method comprises providing the aqueous solution at a flow rate of no greater than approximately 0.5 mL/min. In some embodiments the method comprises providing the aqueous solution at a flow rate of at least 0.1 mL/min. The flow rate of the aqueous solution may be from approximately 0.1 mL/min to approximately 0.3 mL/min, for example approximately 0.2 mL/min.

The total volume of aqueous solution flowed to and through the chamber may be from 0.1 to 1 mL, e.g. from 0.1 to 0.5 mL, for example from 0.2 to 0.4 mL, e.g. 0.3 mL.

In some embodiments, the chamber has a total volume capacity of no greater than approximately 50 µL, for example no greater than approximately 40 µL, for example no greater than approximately 30 µL, for example no greater than approximately 20 µL, for example no greater than approximately 15 µL, for example no greater than approximately 10 µL, for example no greater than approximately 8 µL. A high ratio of the solution volume to cell volume is believed to improve the efficiency of release of the radioisotope.

The chamber may form a part of a microfluidic cell. Aptly, the use of a microfluidic cell enables the manipulation of low volumes and is thus beneficial in that it generates less waste, reduces reagent costs and requires lower sample volumes compared to known batch processes. A microfluidic cell additionally enables faster response times due to short diffusion distances, small heat capacities and better process control due to the faster response of the system. A microfluidic cell is thus particularly suited to the dose-on-demand production of radiopharmaceuticals.

In one embodiment, the microfluidic cell may form a part of a microfluidic system. The microfluidic system may be configured for the synthesis, purification and/or analysis of a radiopharmaceutical. The microfluidic cell may be in fluid communication with one or more other components of the system. In some embodiments, the microfluidic system comprises the microfluidic cell and at least one of a radiotracer production module and a quality control module.

In some embodiments, the microfluidic cell is comprised in or constitutes a Radioisotope Isolation Module (RIM) configured to receive an aqueous solution comprising a radioisotope.

In some embodiments, the microfluidic system comprises a Radioisotope Isolation Module (RIM), wherein the microfluidic cell is comprised in or constitutes the Radioisotope Isolation Module (RIM) and is configured to receive an aqueous solution comprising a radioisotope.

In some embodiments, the microfluidic system comprises:
a) a Radioisotope Isolation Module (RIM) as described herein; and
b) a Radiopharmaceutical Production Module (RPM) configured to receive a first sample which comprises a concentrated and activated radioisotope from the RIM.

In some embodiments, the microfluidic system comprises:
a) a Radioisotope Isolation Module (RIM) as described herein;
b) a Radiopharmaceutical Production Module (RPM) configured to receive a first sample which comprises a concentrated and activated radioisotope from the RIM; and
c) a Purification Module (PM) configured to receive a second sample which comprises a radiopharmaceutical and one or more further components from the RPM.

In some embodiments, the microfluidic system comprises:
a) a Radioisotope Isolation Module (RIM) as described herein;
b) a Radiopharmaceutical Production Module (RPM) configured to receive a first sample which comprises a concentrated and activated radioisotope from the RIM;
c) a Purification Module (PM) configured to receive a second sample which comprises a radiopharmaceutical and one or more further components from the RPM; and
d) a Quality Control Module (QCM), configured to receive a third sample from the PM which comprises a purified radiopharmaceutical and which is further configured to determine one or more characteristics of the third sample, wherein each of the modules is a microfluidic component.

Aptly, the integrated system and the RIM, RPM, PM and QCM are provided on a unitary microfluidic device.

Aptly, the Radiotracer Production Module (RPM) may comprise a micromixer and/or a microreactor.

The microfluidic cell and/or system may be comprised on a microfluidic chip.

In one embodiment, the first electrode may also be referred to as the working electrode. Aptly, the first electrode comes into contact with the flow of aqueous solution.

The first electrode is formed from a carbon rod or section thereof and has a flat surface comprising a plurality of recesses. Aptly, circular and rectangular electrodes have been shown to have excellent performance in both trapping and release of a radioisotope.

In some embodiments, the first electrode is formed from graphite. In some embodiments, the first electrode is formed from glass carbon. In some embodiments, the first electrode may be formed by slicing a thin section from a commercially available carbon rod, such as those sold by Goodfellow Cambridge Ltd. Aptly, the carbon rod is made of graphite but is hard, unlike normal graphite which is soft. Thus, in some embodiments the first electrode is not formed from standard graphite e.g. a layered graphite.

In some embodiments, the first electrode has a hardness of more than 1.0, for example more than 1.1, for example more than 1.2, for example more than 1.3, for example more than 1.4, for example more than 1.5, for example more than 1.6, for example more than 1.7, for example more than 1.8, for example more than 1.9 and/or for example more than 2.0 on the Mohs scale.

In certain embodiments, the first electrode has a hardness of more than 2.0 on the Mohs scale e.g. 2.1 or greater, e.g. 2.2 or greater, e.g. 2.3 or greater.

SEM images have shown that, unlike soft graphite which has a layered structure, a section of carbon rod has a flat surface comprising a plurality of recesses. Thus, the first electrode comprises an irregular surface. In use in the method of the present invention, this irregular surface comes into contact with the flow of aqueous solution. This surface structure of the carbon rod is believed to be favourable for trapping and release of a radioisotope. It has also been found that, unlike soft graphite, the first electrode formed from the carbon rod is not deformed after trapping of a radioisotope, thereby significantly increasing the performance of the electrode. Thus, the first electrode is not deformed in use. In further embodiments, the first electrode is capable of retaining its original form when a voltage of at least 0.5 V, for example at least 1 V, for example at least 3 V, for example at least 5 V, for example at least 10 V, for example at least 15 V or for example at least 20 V is applied across the electrodes.

In some embodiments, the carbon rod has a purity of at least 99%, for example at least 99.5%, for example at least 99.8% or for example at least 99.9%. The carbon rod may comprise trace amounts of other elements such as, but not limited to, Al, B, Ca, Cu, Fe, Mg, Na and/or Si. Each of these elements may be present in an amount of no greater than 1%, no greater than 0.5%, no greater than 0.1%, no greater than 0.05% or no greater than 0.01%.

A circular first electrode may be formed by cutting a section of a desired thickness from a carbon rod. If a non-circular electrode is required, the section can be shaped into any suitable shape (e.g. rectangular) using sand paper. A first electrode produced in this way may be referred to as a carbon disk.

The first electrode may have a polished surface layer. This polished surface layer may come into contact with the flow of aqueous solution. Polishing is thought to clean the electrode surface and reduce the emission of particulates during use which can be adversely affect the performance of the electrode.

The first electrode may have a thickness of from 0.1 mm to 0.5 mm, for example, from 0.2 mm to 0.3 mm, e.g. 0.25 mm.

In some embodiments, the surface area of the first electrode which comes into contact with the flow of aqueous solution is at least 10 mm², at least 15 mm², at least 20 mm², at least 30 mm², at least 40 mm², at least 50 mm², at least 60 mm² or at least 70 mm². In some embodiments, the surface area of the first electrode which comes into contact with the flow of aqueous solution is no greater than 100 mm², no greater than 90 mm², no greater than 80 mm², no greater than 75 mm², no greater than 55 mm², no greater than 35 mm² or no greater than 25 mm².

The second electrode may also be referred to as the counter electrode. The second electrode may be formed from a transition metal. Suitable transition metals include for example gold, silver, iron, zinc, copper and platinum. In some embodiments, the second electrode is formed from platinum, for example platinum foil. Platinum is particularly suitable due to its electrochemical stability.

In some embodiments, the distance between the first and second electrodes is no greater than 0.5 mm. In some embodiments, the distance between the first and second electrodes is at least 0.1 mm. In some embodiments, the distance between the first and second electrodes is from 0.1 to 0.5 mm, for example from 0.2 mm to 0.4 mm, for example from 0.25 mm to 0.3 mm. Aptly, the smaller the gap between the electrodes, the less the resistance, allowing a relatively low voltage to be used.

In some embodiments, the first electric field is generated by applying a voltage of no greater than 50 V, for example no greater than 30 V, or for example no greater than 20 V across the first and second electrodes. In some embodiments, the first electric field is generated by applying a voltage of from 5 to 20 V, for example 10 to 20 V, e.g. from 14 to 20 V, across the electrodes. When the first electric field is generated between the electrodes, the first electrode will have a positive charge (i.e. the first electrode functions as the anode) such that negatively charged radioisotope ions present in the aqueous solution are attracted to the first electrode, causing them to be trapped on the first electrode.

In certain embodiments, the efficiency of trapping of the radioisotope on the first electrode may be at least 94%.

In some embodiments, the method comprises removing the aqueous solution from the chamber prior to providing the flow of the organic-based solution into the chamber.

The organic-based solution may comprise an organic solvent. Suitable organic solvents include acetonitrile, DMF, DMSO and N-methylformamide. Because some radioisotope ions such as ¹⁸F are insoluble in organic solvents, the organic-based solution may further comprise a phase transfer reagent. In some embodiments, the phase transfer reagent is kryptofix 222 (K222). The concentration of K222 may be from 0.027 to 0.08 M.

Aptly, the organic solution further comprises a source of counter ions. Aptly, if the radioisotope ions are negatively charged, the counter ions will be positively charged. The counter ions may be alkali metal ions, such as sodium or potassium ions. In some embodiments, the organic solution comprises a source of potassium ions, such as K₂CO₃ or KHCO₃. The concentration of K₂CO₃ or KHCO₃ may be from 0.01 to 0.04 M.

In some embodiments, the organic solution further comprises from 1 to 10%, e.g. from 2 to 8 %, aptly from 3 to 6% H₂O by volume. In some embodiments, the H₂O content of the organic solution is about 4%. Aptly, a small amount of water significantly increases the solubility of the insoluble radioisotope ions in the organic-based solution, thereby facilitating the release of the radioisotope from the first electrode. A water content of greater than 10% may have a detrimental effect on subsequent reactions of the released radioisotope.

In some embodiments the method comprises providing the organic-based solution at a flow rate of at least 1 mL/min. The flow rate may be from 0.05 to 1 mL/min, for example 0.1 to 1 mL/min.

In some embodiments the method comprises providing the organic-based solution at a flow rate of at least 0.05 mL/min. The flow rate may be from 0.05 to 0.5 mL/min, or example from 0.08 to 0.3 mL/min, for example from 0.1 to 0.2 mL/min.

In some embodiments, the method comprises providing the aqueous solution at a higher flow rate i.e. at a greater flow rate than the flow rate that the organic-based solution is provided at.

Aptly, the method comprises providing the aqueous solution at a flow rate of approximately 0.2 mL/min and the organic-based solution at a flow rate of approximately 0.1 mL/min.

The total volume of the organic-based solution provided to the chamber may be from about 0.1 to 0.5 mL, for example from about 0.2 to 0.4 mL, e.g. 0.3 mL.

In some embodiments, the method further comprises washing the chamber prior to providing the flow of an organic-based solution through the chamber. The wash step may be carried out after trapping the radioisotope on the first electrode. Washing may be carried out by flowing an organic solvent, or a solution comprising an organic solvent, through the chamber. The organic solvent may be the same as that comprised within the organic-based solution which is subsequently flowed into the chamber before release of the trapped ions is effected. The washing step helps to remove residual aqueous solution from the chamber. In some embodiments, the chamber is washed with acetonitrile.

In some embodiments the second electric field is generated by applying a voltage of no greater than 20 V for example no greater than 10 V, e.g. no greater than 5 V across the first and second electrodes. In some embodiments the second electric field is generated by applying a voltage of from about 1 to 5 V, for example from about 1.5 to 4.5 V (e.g. from 1.6 to 4.1 V) across the electrodes. It will be understood that the voltages described herein in relation to the second electric field may also be expressed as negative values (e.g. -10 V or - 5 V), since the second electric field has an opposite polarity to the first electric field. When the second electric field is generated, the first electrode gains a negative charge (i.e. it becomes the cathode). This causes the trapped radioisotope ions to be repelled and released from the first electrode.

The trapping of radioisotope ions from an aqueous solution on an electrode, followed by release of the ions into an organic-based solution as provided by certain embodiments of the present invention thus offers a convenient method of solvent exchange which is simpler, faster and easier to automate than conventional techniques based on anion exchange and azeotropic evaporation.

Thus, the method of the certain embodiments of the invention may also be for concentrating the radioisotope.

The efficiency of release may be at least 90%, for example at least 95%, for example at least 96%, for example at least 97% or for example at least 98%.

In some embodiments, the method further comprises heating the chamber and/or the organic-based solution. Heating may be carried out by placing the chamber e.g. within a microfluidic cell on a hotplate. Heating may be carried out prior to generating the second electric field. It has been found that heating can facilitate the release of the radioisotope from the second electrode, particularly when using small volumes of solution. The chamber and/or organic-based solution may be heated to a temperature of from 40 to 100 °C, e.g. 50 to 100 °C, e.g. 40 to 80 °C, for example 40 to 60°C , or for example from 60 to 90 °C or for example from 70 to 80 °C.

In some embodiments, the chamber may be heated indirectly. For example, where the chamber is part of an integrated system, for example as part of a microfluidic system comprised on a microfluidic chip, it may not be necessary to place the chamber directly adjacent to or in direct contact with a heat source in order to heat the chamber. Heating may be achieved indirectly through heat transfer by heating another area of the integrated system, for example by heating another area of the microfluidic chip.

Aptly, the method may further comprise reacting the radioisotope released from the second electrode with a precursor to provide a radiopharmaceutical or a radiotracer or an intermediate in the synthesis of a radiopharmaceutical.

It will be appreciated that the precursor will be selected in accordance with the desired radiotracer. The radiotracer may be any compound which is prepared by nucleophilic substitution using ¹⁸F. Examples of radiotracers include [¹⁸F]-FDG, 3'-deoxy-3'-(18)F-fluorothymidine (FLT), FMISO and sodium fluoride.

In embodiments wherein the desired radiotracer is [¹⁸F]-FDG, the precursor is mannose triflate. Suitable precursors for the preparation of other common radiolabelled substances will be known by those skilled in the art.

In some embodiments, the method comprises transferring the organic-based solution comprising the released radioisotope to a reactor where the radioisotope is reacted with the precursor. The reactor may form a part of the microfluidic cell or a separate device.

In some embodiments, the method further comprises transferring the organic-based solution comprising the released radioisotope to a mixer where the radioisotope is mixed with the precursor. The mixture may then be transferred to a reactor where the reaction between the radioisotope and the precursor is effected, for example by heating.

In some embodiments, a microfluidic system comprises a RIM and a RPM. The RIM comprises the chamber. The RPM comprises a microreactor and/or a micromixer. Where the organic-based solution comprising the released radioisotope is for transfer from the RIM to the RPM, heating of the chamber may be achieved by heating part or all of the RPM directly. The chamber is not heated directly but is heated indirectly through heat transfer from the RPM. The RPM may comprise a reactor. The reactor may be heated directly. The RPM may comprise a mixer. The mixer may be heated directly.

Alternatively, the released radioactive isotope may be reacted with the precursor in the chamber. In some embodiments, the organic-based solution may additionally comprise the precursor such that the radioisotope reacts with the precursor in the chamber upon release of the radioisotope from the first electrode. Aptly, the cell may comprise one or more additional inlets for providing the precursor.

The method may further comprise additional chemical processing steps which may be necessary for obtaining the final radiopharmaceutical. The additional chemical processing steps may include hydrolysis, deprotection and/or purification. For example, in embodiments wherein the radioisotope is [¹⁸F]-fluoride and the precursor is mannose triflate, the nucleophilic substitution reaction generates acetylated [¹⁸F]-FDG which must then be hydrolysed to remove the protecting groups in order to produce the final radiotracer [¹⁸F]-FDG. The appropriate chemical processing steps required for the synthesis of a desired radiopharmaceutical will be apparent to those skilled in the art.

The overall yield of radiolabelled substances prepared in accordance with the methods of certain embodiments of the invention may be at least 90% or at least 95%.

In some embodiments, a method for separating and recovering a radioisotope from an aqueous solution comprising the radioisotope comprises using a microfluidic device comprising a chamber of a microfluidic cell:
flowing the aqueous solution to the chamber of the microfluidic cell, the chamber comprising a first electrode and a second electrode;
generating a first electric field between the first and second electrodes, thereby trapping the radioisotope on the first electrode;
flowing an organic-based solution to the chamber comprising the first and the second electrodes;
generating a second electric field between the first and the second electrodes, wherein the second electric field has an opposing polarity to the first electric field, thereby releasing the radioactive isotope from the first electrode into the organic based solution,
wherein the first electrode is formed from a carbon rod or section thereof and has a flat surface comprising a plurality of recesses,
wherein the chamber has a volume of no greater than approximately 40 µl,
wherein the first electric field is generated by applying a voltage of no greater than 30 V across the first and second electrodes,
wherein the second electric field is generated by applying a voltage of no greater than 5 V across the first and second electrodes,
optionally, wherein the organic-based solution comprises from 1 to 10% water;
optionally, wherein the first electrode has a polished surface layer;
optionally, wherein the flow rate of the aqueous solution is from approximately 0.1 mL/min to approximately 0.3 mL/min;
optionally, wherein the flow rate of the organic-based solution is from approximately 0.1 mL/min to approximately 0.2 mL/min;
optionally, wherein the surface area of the first electrode which comes into contact with the flow of aqueous solution is at least 20 mm²; and
optionally, wherein the method further comprises heating the chamber and/or the organic-based solution to a temperature of from 40 to 100 °C, e.g. 50 to 100 °C, e.g. 40 to 80 °C prior to applying the second electric field.

In some embodiments, the apparatus is a microfluidic cell.

In some embodiments, the apparatus comprises a pair of opposing plates. The plates may support the first and second electrodes in a face-to-face arrangement. The plates may be formed from any suitable material such as glass, quartz, silicon or ceramics, or a polymer such as polymethylmethacrylate (PMMA), polycarbonate, polydimethylsiloxane (PDMS), polydicyclopentadiene (DCPD) and polyetheretherketone (PEEK). In some embodiments, the plates are made of glass. The plates may be machined or moulded to the required size and shape.

In some embodiments, the plates are fixed together. The plates may be fixed together so as to provide a liquid-tight seal around the chamber. Any suitable means may be used to fix the plates together, including screws, adhesives, double-sided tape, ultrasonic bonding or thermal bonding. It will be appreciated that the type of fixing that is appropriate will depend on the material from which the plates are made. For plastic plates, double-sided tape, adhesives or ultrasonic bonding may be used. For glass plates, thermal bonding may be most suitable. The plates may be in direct contact in the assembled cell, or they may be spaced apart.

In some embodiments, the apparatus further comprises a spacer or gasket between the opposing plates. The spacer may be formed from any suitable material. In some embodiments, the spacer is formed from silicon.

A chamber may be defined, at least in part, by a cavity, channel or cut-out in one or both plates and/or in the spacer. In some embodiments, the chamber is defined by a channel in the spacer. The channel may be straight, or it may be serpentine.

In some embodiments, the apparatus is configured to receive fluid at a flow rate of at least 1 mL/min, at least 0.05 mL/min, at least 0.1 mL/min, at least 0.2 mL/min or at least 0.3 mL/min.

In some embodiments, the chamber has one or more side walls, a floor and a ceiling. The side wall(s) of the chamber may be formed by the plate(s) and/or the spacer. A part or the whole of the floor of the chamber may be formed by one of the first and second electrodes. A part or the whole of the ceiling of the chamber may be formed by the other of the first and second electrodes.

The chamber may be any suitable size or shape. In some embodiments, the chamber has a rectangular cross-section. In other embodiments, the chamber is coin-shaped.

The apparatus may further comprise leads which connect the electrodes to a circuit. One or more holes may be provided in one or both plates to enable electrical connection with the electrodes.

In some embodiments, the inlet is provided by a hole or a channel in one of the pair of opposing plates. The outlet may be provided by a hole or a channel in the same plate in which the inlet is provided, or in the other of the pair of opposing plates. Aptly, the cell may comprise one or more further inlets.

In some embodiments, the apparatus further comprises a heater. The heater may be configured for heating the chamber, or it may be configured for heating fluid before the fluid enters the chamber. In some embodiments, the heater is configured for indirect heating of the chamber. In some embodiments, the heater comprises a hot plate, a heating element or a heat mat. In some embodiments, the chamber, or the entire apparatus or microfluidic cell, is positioned on the heater. The heater may be manually or automatically operable.

In some embodiments, the invention provides a microfluidic system comprising:
a) a RIM as described herein, for example the RIM comprising an apparatus for separating and recovering a radioisotope from an aqueous solution comprising the radioisotope, the apparatus comprising:
   an inlet;
   an outlet; and
   a chamber in fluid communication with the inlet and the outlet to form a fluid pathway, wherein the chamber comprises a first electrode formed from a carbon rod or section thereof and having a flat surface comprising a plurality of recesses and a second electrode; and
b) a RPM, the RPM comprising a micromixer and/or a microreactor.

Aptly the RIM constitutes or comprises a microfluidic cell of the invention.

The microfluidic system may further comprise a heater configured to heat all or part of the RPM. Aptly the heater may be configured to heat the micromixer and/or the microreactor. Aptly heating all or part of the RPM results in heat transfer to the chamber of the RIM. In some embodiments, the heater comprises a hot plate, a heating element or a heat mat. The heater may be manually or automatically operable.

Temperature plays an important role in the reaction rate and efficiency of chemical and electrochemical processes. Where one or more of these processes occurs within an integrated system, for example within a microfluidic system (e.g. in an integrated microfluidic chip), it may be advantageous to control and/or differentiate the temperature at which each process occurs. For example, it may be advantageous if the chamber is maintained at a temperature which is lower than that of all or part of the RPM.

Aptly, the microfluidic system may further comprise a heat reduction means. The heat reduction means operates to reduce heat transfer from the RPM to the chamber when the RPM is heated directly. In some embodiments, the heat reduction means is a slot formed in the microfluidic system (e.g. in a microfluidic chip comprising the RIM and the RPM) between the chamber and all or part of the RPM.

In some embodiments, there is a temperature difference of greater than 50°C between the directly heated area of the RPM and the chamber, particularly when the RPM is heated to temperatures in excess of 90°C, for example to approximately 100°C.

In some embodiments, an apparatus for separating and recovering a radioisotope from an aqueous solution comprising the radioisotope comprises a microfluidic cell comprising:
an inlet;
an outlet; and
a chamber in fluid communication with the inlet and the outlet to form a fluid pathway, wherein the chamber comprises a first electrode and a second electrode,
wherein the first electrode is formed from a carbon rod or section thereof and has a flat surface comprising a plurality of recesses,
wherein the chamber has a volume of no greater than approximately 40 µL,
optionally, wherein the first electrode has a polished surface layer,
optionally, wherein the distance between the first electrode and the second electrode is no greater than 0.5 mm,
optionally, wherein the surface area of the first electrode which comes into contact with the flow of aqueous solution is at least 20 mm²;
optionally wherein the apparatus is a microfluidic device e.g. a microfluidic chip or cell

In certain embodiments, the system further comprises a holder device for holding a microfluidic chip comprising a microfluidic cell. The holder device may comprise a holder body having a cavity configured to hold and secure the chip. Aptly, the holder body comprises an upper layer and a lower layer. Aptly, the upper layer comprises an upper surface which comprises one or more through holes configured to accommodate one or more connecting elements. Aptly, at least one of the one or more through holes is configured to accommodate an optical fibre and/or a wire element. Aptly, each through hole is configured to enable the connecting element to connect the chip to further downstream elements for example to a detector and/or a source to a detection zone of the chip.

The apparatus of the present invention is simple and cheap to fabricate, and enables electro- trapping and release of radioisotopes with high efficiency. The compact design conveniently enables fast and safe dose-on-demand production of radiopharmaceuticals. The use of a microfluidic cell circumvents the need for azeotropic evaporation, thereby significantly shortening production time.

### Detailed description of the invention

Certain embodiments of the present invention relate to a microfluidic cell, a microfluidic chip and system comprising such a cell and/or chip. A fluid flow can be described as "microfluidic" (i.e. "microfluidic fluid flow") if a fluid passes through a channel having at least one dimension of less than 1mm, in particular a channel having a dimension of less than 1mm, e.g. less than 500µm, less than 250µm, less than 200 µm, or less than 150 µm. This creates laminar flow characteristics (generally having a Reynolds number of less than 100) where diffusion is the dominant cross stream chemical interaction. Consequently, microfluidic fluid flow occurs during the manipulation of small volumes, for example from 1µm to 100µl, within microstructured devices that features dimensions of the order of 10's to 100's micrometers.

In certain embodiments, a "microfluidic flow system" comprises a system having at least one channel for fluid flow, the channel having at least one dimension of less than one 1mm, for example less than 500µm, e.g. less than 300 µm, e.g. less than 200 µm, e.g. less than 150 µm, e.g. less than 100 µm, e.g. less than 50 µm. The microfluidic flow system comprises a microfluidic device but may also comprise other components that are in fluid communication with the microfluidic device.

In one embodiment, the system comprises one or more channels having a width of, for example, between about 100 µm to about 200 µm e.g. about 150 µm and a depth of for example about 50 µm deep.

A "microfluidic chip" or a "microfluidic cell" can be identified by the fact that it has one or more channels with at least one dimension less than 1 mm, for example, less than 500µm, e.g. less than 300 µm, e.g. less than 200 µm, e.g. less than 150 µm, e.g. less than 100 µm, e.g. less than 50 µm, in particular a channel having a dimension of less than 1mm, e.g. for example, between about 100 µm to about 200 µm e.g. a width of about 150 µm and a depth of between about 40 to 60 µm, for example about 50 µm. The microfluidic chip may be part of a microfluidic flow system. The one or more channels may form a fluid flow path in the chip or cell.

As used herein, a "radiopharmaceutical" is an isotopically labelled analogue of a pharmaceutical molecule. Aptly, a radiopharmaceutical can be used for diagnostic or therapeutic purposes.

As used herein, a "radiotracer" is a radiopharmaceutical having a largely unaltered metabolic pathway compared to the unlabelled analogue. It is therefore possible to follow and quantify processes on a particular metabolic pathway by detecting the radioactive decay of the labelling radioisotope. Radiotracers are used for diagnostic purposes. Examples of radiotracers include, but are not limited to, ¹⁸F-FLT ([¹⁸F]fluoro thymidine), ¹⁸F-FDDNP (2-(I-{ 6-[(2-[¹⁸F]fluoroethyl)(methyl)amino]2-naphthyl}ethylidene)malonitrile), ¹⁸F-FHBG (9-[4-[¹⁸F]fluoro-3-(hydroxymethyl)butyl]guanine or [¹⁸F]-penciclovir), ¹⁸F-FESP ([¹⁸F]-fluoroethylspiperone), ¹⁸F-p-MPPF(4-(2-methoxyphenyl)-I-[2-(N-2-pyridinyl)-p[¹⁸F]fluorobenzamido]ethylpiperazine), ¹⁸F-FDG (2-[¹⁸F]fluoro-2-deoxy-D-glucose), ¹⁸F-FMISO (¹⁸F-fluoromisonidazole) and ¹⁸F-sodium fluoride.

Embodiments of the invention will now be described by way of example only and with reference to the accompanying Figures in which:
Figure 1 is an exploded view of a microfluidic cell in accordance with an embodiment of the present invention;
Figure 2 is a perspective view of the microfluidic cell of Figure 1;
Figure 3 is an exploded view of a microfluidic cell in accordance with another embodiment of the present invention;
Figure 4a is a schematic side view of a microfluidic cell in accordance with a further embodiment of the present invention;
Figure 4b is an exploded view of the microfluidic cell of Figure 4a;
Figure 5 is a schematic diagram of a microfluidic system comprising the microfluidic cell of Figure 4; and
Figure 6a depicts a top view of a microfluidic system comprising a RIM and RPM as described herein which comprises a heat reduction means; and
Figure 6b illustrates the temperature difference observed between the directly heated area of the RPM and the chamber of the RIM as measured with an infrared thermometer;
Figure 7A-C illustrate the microfluidic system of Figure 6 secured in a holder.

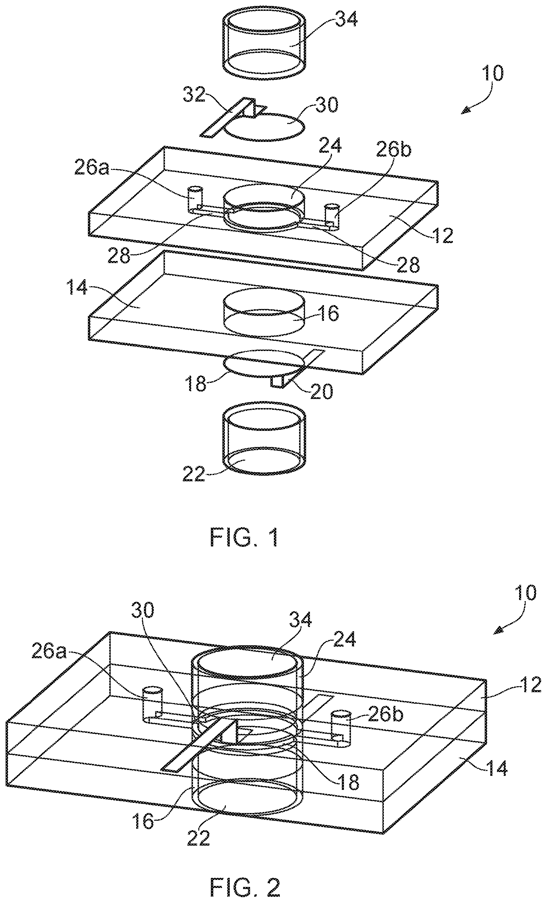

With reference to Figure 1, a microfluidic cell 10 comprises upper 12 and lower 14 rectangular glass plates, which have been machined to the desired size and shape. It will be appreciated that in alternative embodiments, the plates may be formed from other materials such as quartz or polymer. In the centre of the lower plate 14 there is a circular cut-out 16 which extends through the entire depth of the plate 14, and which receives a carbon disk (10 mm diameter) that forms the working electrode 18. The working electrode 18 is cut to size and polished. A first lead 20 connects the working electrode 18 to an electrical circuit (not shown). The working electrode 18 is secured in place by a first cylindrical glass liner 22 which is sized to fit into the cut-out 16 of the lower plate 14. The glass liner 22 may be a stock component, or it may be machined to the required shape and size.

The upper plate 12 has a circular cut-out 24 in a position corresponding to that of the cut-out 16 in the lower plate 14. The upper plate further comprises two holes 26, one either side of the cut-out 24, each of which is fluidly connected to the cut-out 26 via a channel 28. One of the holes 26a provides an inlet to allow fluids into the cell 10, while the other 26b provides an outlet. A circular platinum counter electrode 30 (10 mm diameter) is received in the cut-out 24 in the upper plate 12. The counter electrode 30 is connected to the circuit by a second lead 32. The counter electrode 30 is secured in place by a second cylindrical glass liner 34 which is sized to fit into the cut-out 24 in the upper plate 12. The glass liner 34 may be a stock component, or it may be machined to the required shape and size.

Figure 2 shows the cell 10 of Figure 1 with the components assembled. To assemble the cell, the upper and lower plates 12, 14 are aligned before being thermally bonded together. Once the plates 12, 14 are bonded the counter electrode 30 is placed within the cut-out 24 in the upper plate 12. The electrode lead 32 is inserted and a sealant is applied to the interior surface of the cut-out 24 before inserting the second glass liner 34. The sealant is allowed to set before the process is repeated for securing the working electrode 18 in the cut-out 16 in the lower plate 14.

Once assembled, the upper and lower plates 12, 14 together with the first and second glass liners 22, 34 define a chamber which houses the working electrode 18 and the counter electrode 30 in a face-to-face arrangement. The gap between the electrodes is 250 µm, and the volume of the chamber is 19.6 µL. Fluids can be flowed through the chamber via the inlet and outlet 26a, 26b.

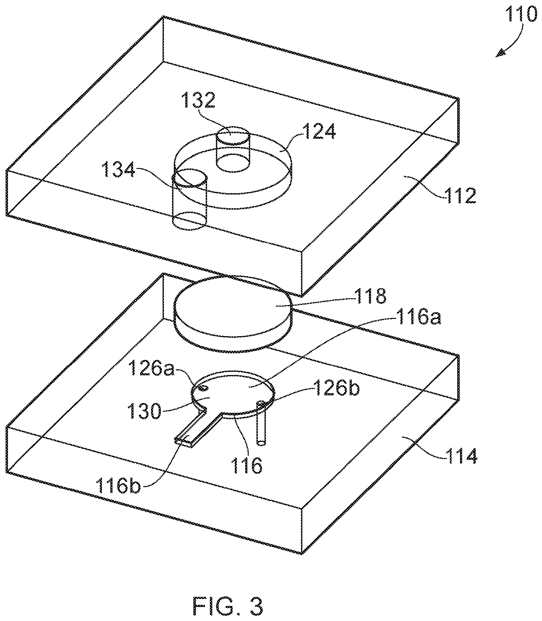

Figure 3 shows an alternative embodiment of a microfluidic cell 110 comprising upper 112 and lower 114 glass plates which are substantially square. The lower plate 114 comprises a cavity 116 which provides a chamber for electro-trapping and release. The cavity 116 can be any depth or shape. In the embodiment shown, the cavity 116 is key-shaped, i.e. it comprises a circular portion 116a and an elongate portion 116b extending from the circular portion 116a. A sputter coated metallic layer formed on a surface of the cavity 116 provides the counter electrode 130. Two holes 126 extend through the lower plate 114 into the cavity 116, providing an inlet 126a and an outlet 126b for fluids.

The microfluidic cell 110 further comprises a disk-shaped working electrode 118 formed from a carbon rod. The working electrode 118 is greater in diameter than the circular portion 116a of the cavity 116 in the lower plate 114 which forms the chamber. The working electrode 118 is housed within a circular recess 124 in the upper plate 112. The upper plate 112 further comprises a first hole 132 in the approximate centre of the plate which extends into the recess 124, enabling electrical connection of the working electrode 118. A second hole 134 in the upper plate 112 is provided in a position corresponding to the elongate portion 116b of the cavity 116 in the lower plate 114, thereby enabling electrical connection with the counter electrode 130.

To assemble the cell 110, the upper plate 112, lower plate 114 and working electrode 118 are loosely assembled and the plates 112, 114 aligned before the plates 112, 114 are thermally bonded together. The electrical contacts (not shown) are then secured in the holes 132, 134 in the upper plate 112 using a sealant

With reference to Figures 1-3, an embodiment of a method of radioisotope recovery (solvent exchange) by electro-trapping and release using a microfluidic cell in accordance with the present invention will now be described. The method first comprises flowing ¹⁸O-enriched water containing ¹⁸F into the chamber of the microfluidic cell 10, 110 via the inlet 26a, 126a. An electric field is generated between the electrodes 18, 118, 30, 130 by applying a voltage across the electrodes such that the negatively charged ¹⁸F ions are attracted to the working electrode 18, 118, causing them to be trapped on the working electrode 18, 118. Waste H₂ ¹⁸O is removed from the cell 10, 110 via the outlet 26b, 126b. The chamber is then washed by flowing acetonitrile through the chamber. An organic-based solution comprising K222 and K₂CO₃ in acetonitrile (and optionally 4% H₂O) is introduced into the chamber via the inlet 26a, 126a. The polarity of the electric field is then reversed, causing the ¹⁸F ions to be released from the working electrode 18, 118 into the organic-based solution. The organic-based solution containing the released ¹⁸F ions can then be removed from the chamber via the outlet 26b, 126b, and transferred to a separate reactor where the ¹⁸F ions are reacted with a precursor to generate a radiotracer. Alternatively, the precursor may be provided in the organic-based solution so that the nucleophilic substitution reaction between the precursor and the ¹⁸F ions occurs within the chamber.

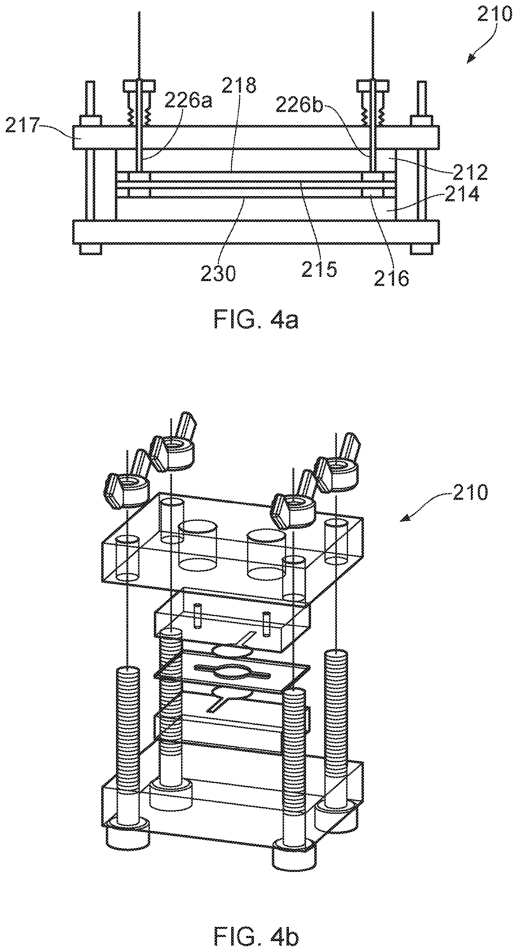

Turning to Figure 4a, in a further embodiment a microfluidic cell 210 comprises a rectangular upper plate 212, an opposing lower plate 214 of the same size and shape as the upper plate 212, and a silicon spacer 216 disposed between the upper and lower plates 212, 214. The plates 212, 214 are machined to the required size and shape. The upper and lower plates 212, 214 and the silicon spacer 216 are held together within a clamp 217. A cut-out is formed in the centre of silicon spacer 216, thus defining a channel which constitutes a reaction chamber 215. A rectangular working electrode 218 formed from a section of carbon rod is positioned against the upper plate 212 and forms a ceiling of the reaction chamber 215. The working electrode 218 is polished prior to assembly of the cell 210. A platinum foil counter electrode 230 is positioned on the lower plate 214, facing the working electrode 218, forming a floor of the reaction chamber 215. The electrodes are held in place by the force of the clamp 217 and are sealed using sealant. Leads connect the working and counter electrodes 218, 230 to a circuit. Two holes 226 are formed in the upper plate which provide an inlet 226a and an outlet 226b to allow fluids to flow through the chamber 215 via capillary tubing. Figure 4b shows an exploded view of the microfluidic cell 210.

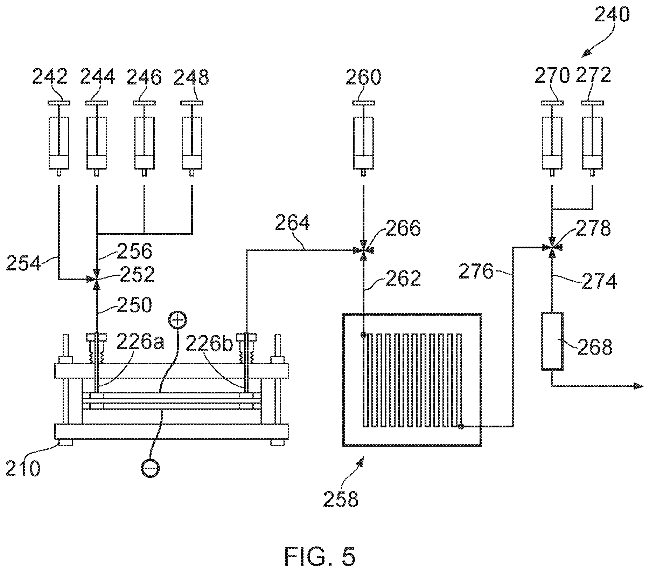

Figure 5 shows a microfluidic system 240 for the preparation of a radiotracer. In the embodiment shown, the system is set up for the synthesis of [¹⁸F]-FDG, but it will be appreciated that the system could be used to prepare any desired radiopharmaceutical. The system eliminates evaporation steps for solvent exchange, and enables dose-on-demand production of radiotracers in an integrated system.

The system 240 comprises the microfluidic cell 210 for solvent exchange of ¹⁸F by electro-trapping and release. The microfluidic cell 210 is supplied with fluids from a set of four syringes, a first syringe 242 containing a source of ¹⁸F, a second syringe 244 containing acetonitrile for washing, a third syringe 246 containing an organic-based solvent and a fourth syringe 248 containing water. A line 250 connects the syringes 242, 244, 246, 248 to the inlet 226a of the microfluidic cell 210. The flow of fluids into the cell 210 is controlled by a first valve 252 positioned at a point at which the line 250 diverges between a first branch 254 leading to the first syringe 242 and a second branch 256 leading to the second 244, third 246 and fourth 248 syringes.

The system further comprises a microfluidic reactor 258 and a fifth syringe 260, which contains mannose triflate. A flow path 262 connects the fifth syringe 260 to the microfluidic reactor 258. A line 264 feeds fluids from the outlet 226b of the microfluidic cell 210 into the flow path 262. A second valve 266 is positioned at the junction of the line 264 with the flow path 262, and controls the flow of fluids into the microfluidic reactor 258.

The system additionally comprises a C₁₈ deprotection column 268, a sixth syringe 270 containing water for elution and a seventh syringe 272 containing sodium hydroxide for hydrolysis. A flow path 274 supplies fluids from the sixth and seventh syringes 270, 272 to the deprotection column 268. A line 276 feeds fluids from the microfluidic reactor 258 into the flow path 274. A third valve 278 is positioned at the junction of the line 276 with the flow path 274, and thus controls the flow of fluids into the deprotection column 268.

Recovery of ¹⁸F from an aqueous solution into an organic-based solution by electro trapping and release is carried out using the microfluidic cell 210, as described above. The organic-based solution containing the released ¹⁸F ions leaves the microfluidic cell 210 via the outlet 226b, and is transferred to the microfluidic reactor 258 via the line 264. Mannose triflate is supplied to the microfluidic reactor 258 from the fifth syringe 260 via the flow path 262. Within the microfluidic reactor 258 the mannose triflate undergoes a nucleophilic substitution reaction with the ¹⁸F ions, producing acetylated [¹⁸F]-FDG. The acetylated [¹⁸F]-FDG is then transferred to the deprotection column 268 via line 276 and flow path 274, where it undergoes hydrolysis in the presence of a sodium hydroxide solution to produce [¹⁸F]-FDG.

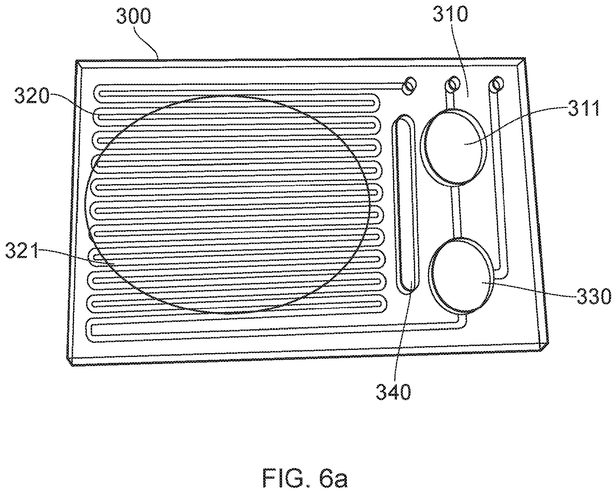

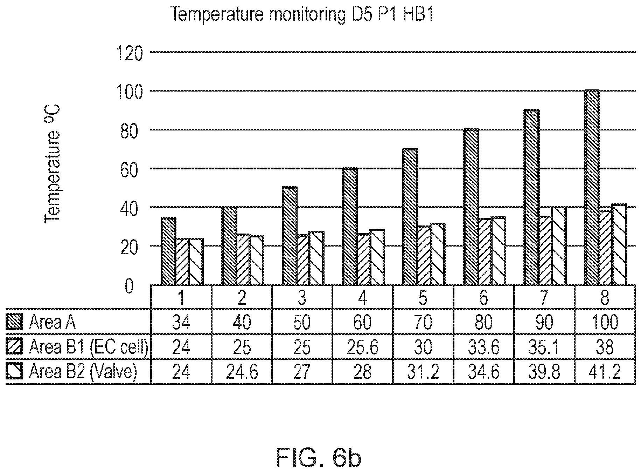

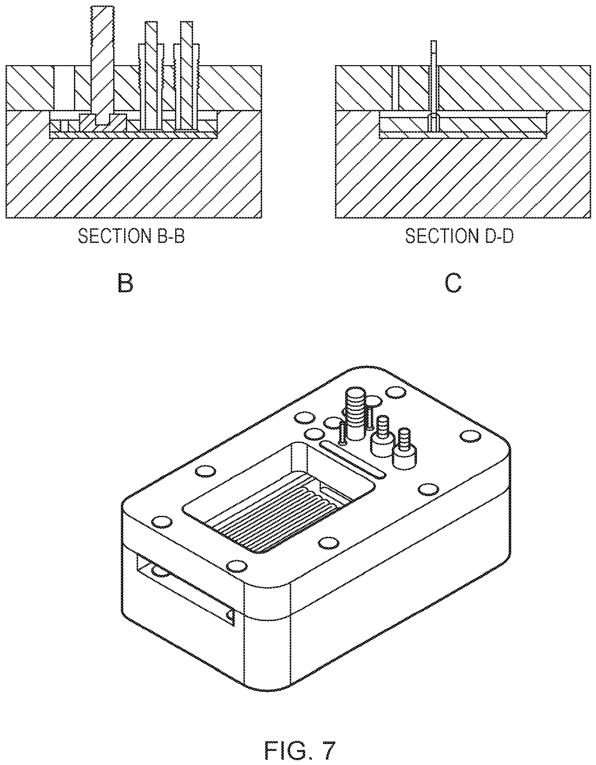

Figure 6a shows a top view of a microfluidic system 300 for the preparation of a radiotracer. In certain embodiments, the system 300 is a combined RIM and RPM as described herein. In the embodiment shown, the system comprises a microfluidic cell 310 comprising a chamber 311 for solvent exchange of ¹⁸F by electro- trapping and release. Adjacent to the microfluidic cell is valve 330. The system further comprises a microfluidic reactor 320. Area 321 of the microfluidic reactor is heated directly by means of a heater (not shown) positioned directly below area 321. Heat reduction means is provided in the form of a slot 340. The slot reduces heat transfer from the directly heated area (321) to the chamber of the microfluidic cell. Figure 7A-C illustrates the microfluidic system of Figure 6 which secured in a holder. The system may further comprise one or more valves which can control flow of solutions within and/or between each module.

Figure 6b is a graphical representation of the temperature difference between area 321 and microfluidic cell 310 as measured by an infrared thermometer following direct heating of area 321.

### Examples

Microfluidic cells similar to that shown in Figure 4 were fabricated by clamping together two glass plates, a pair of electrodes (including a platinum counter electrode) and a silicon spacer comprising a channel. Details of the cell s are provided in Table 1.

The cells were tested with a low volume of ¹⁸O-water comprising ¹⁸F from an ABT cyclotron. Trapping and release efficiencies were probed at different voltages with variation of the flow rate. The results are shown in Tables 2 and 3. Efficiencies of up to 99% in trapping (Table 2) and up to 98% in release (Table 3) were observed.

**Table 1**

| **Cell design** | **Working electrode** | **Cell volume (µL)** |
|---|---|---|
| 1 | Graphite (15x4mm) | 30 |
| 2 | Carbon disk (10 mm diameter) | 32 |
| 3 | Carbon disk (10 mm diameter) | 16 |
| 4 | Carbon 8x3x0.25 mm | 6 |

**Table 2**

| **Cell design** | **Trapping conditions** | | | **Trapping efficiency %** |
|---|---|---|---|---|
| | **Volume ¹⁸O-water (mL)** | **Flow rate (mL/min)** | **Voltage (V)** | |
| 1 | 0.2 | 0.2 | 20 | 99 |
| 2 | 0.3 | 0.2 | 20 | 99 |
| 3 | 0.3 | 0.2 | 15-20 | 98 |
| 4 | 0.3 | 0.2 | 14-20 | 96-99 |

**Table 3**

| **Cell design** | **Release conditions** | | | | | **Release efficiency %** |
|---|---|---|---|---|---|---|
| | **Solvent** | **Volume (mL)** | **Flow rate (mL/min)** | **Voltage (V)** | **Temperature** | |
| 1 | Water | 0.2 | 0.1 | 1.6-3.8 | r.t. | 44-69 |
| 2 | Water-K222 | 0.3 | 0.1 | 1.6-3.8 | r.t. | 94 |
| 2 | K222-K₂CO₃ MeCN-4% H₂O | 0.3 | 0.1 | 1.6-3.8 | r.t. | 85 |
| 2 | K222-K₂CO₃ MeCN | 0.3 | 0.1 | 1.6-4.1 | r.t. | 73 |
| 3 | K222-K₂CO₃ MeCN-4% H₂O | 0.1 | 0.1 | 1.6-4.1 | r.t. | 76 |
| 3 | K222-K₂CO₃ MeCN-4% H₂O | 0.1 | 0.1 | 1.6-4.1 | 80 °C | 98 |
| 4 | K222-K₂CO₃ MeCN-4% H₂O | 0.1 | 0.1 | 1.6-4.1 | r.t. | 82 |
| 4 | K222-K₂CO₃ MeCN-4% H₂O | 0.1 | 0.1 | 1.6-4.1 | 80 °C | 96-98 |

In cell design 1 graphite was used as the anode (the working electrode). A trapping efficiency of 99% was achieved at 20 V within 1 minute but only 44-69% release efficiency could be obtained within 3 minutes. The low release efficiency was thought to be due to deformation of the graphite electrode. The cell was modified by replacing the graphite electrode with an electrode produced by cutting a small piece from a carbon rod (cell design 2, 3 and 3) and a significant improvement in the release efficiency was observed (Table 3). No significant difference in performance of cells 3 and 4 was observed over more than 40 runs, demonstrating excellent stability.

A system similar to that shown in Figure 5 was fabricated and used to synthesise [¹⁸F]-FDG in accordance with the following method. 0.2-0.3 mL of ¹⁸O-enriched H₂O containing ¹⁸F was passed through the cell at a flow rate of 0.2 mL/min under a constant electric potential (14-20 V) applied between the carbon and Pt electrodes. The cell was then washed by flushing with anhydrous MeCN (0.5 mL/min, 1 minute) while the voltage was disconnected. Next, 0.1 mL of organic-based solution containing K222 and KHCO₃ in MeCN-H₂O (1-10%) was passed through the cell at flow rate of 0.1 mL/min under a reversed potential (2-4 V). The cell was heated to a pre-set temperature of 80°C and the released solution was stored in a sample loop. The released solution containing ¹⁸F, K222 and KHCO₃ was pushed by MeCN at flow rate of 0.02 mL/min to mix with 0.1 mL of mannose triflate solution (0.02 mL/min) inside a Y-micromixer. The mixture was then transferred to a microreactor (volume 0.05 mL) heated to 100°C. The reaction solution was mixed with H₂O at a flow rate of 0.04 mL/min and then passed through a C18-monolith column for trapping the labelled precursor. The monolith was washed with water and dried with N₂. 0.4 mL of 2 N NaOH solution was loaded into the monolith and hydrolysis was maintained at room temperature for 2 minutes. The product [¹⁸F]-FDG was eluted out with 1-5 mL of water.

Using this system, ¹⁸F fluoride (initial activity up to 30 mCi) can be trapped with an efficiency of 94-99% and subsequently released with a release efficiency of 96-98%. Using the organic solution containing released ¹⁸F fluoride for fluorination of mannose triflate, up to 100% acetylated [¹⁸F]-FDG. (ACY-[¹⁸F]-FDG) can be obtained. 100% ACY-[¹⁸F]-FDG can be obtained within 1.2 min at 100°C. After basic hydrolysis on the deprotection column (aptly a monolith) 98.3% [¹⁸F]-FDG was obtained. Optionally, the [¹⁸F]-FDG can be further purified by passing through cation-, anion-, silica- and C18-monoliths.

The use of microfluidic cells for processing of low volume of heavy water feed from the cyclotron offers an excellent method for solvent exchange to carry out nucleophilic substitution reactions with standard precursors. In addition to the excellent trap and release performance, the benefits of the invention include the use of a low voltage, re-use of electrodes and simple operation. The present invention thus offers significant potential in efficient dose-on-demand radiotracer production.

Throughout the description and claims of this specification, the words "comprise" and "contain" and variations of them mean "including but not limited to" and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of the features and/or steps are mutually exclusive.

## Claims

1. A method for separating and recovering a radioisotope from an aqueous solution comprising the radioisotope, the method comprising using a microfluidic device (10) comprising a chamber:
flowing the aqueous solution to the chamber, the chamber comprises a first electrode (18) and a second electrode (30);
generating a first electric field between the first and second electrodes, thereby trapping the radioisotope on the first electrode;
flowing an organic-based solution to the chamber comprising the first and the second electrodes; and
generating a second electric field between the first and the second electrodes, wherein the second electric field has an opposing polarity to the first electric field, thereby releasing the radioactive isotope from the first electrode into the organic based solution, wherein the first electrode is formed from a carbon rod or section thereof and has a flat surface comprising a plurality of recesses.

2. The method of claim 1, wherein the method comprises providing a flow of the aqueous solution at a flow rate of at least 0.1 mL/min and/or the method comprises providing a flow of the organic-based solution at a flow rate of at least 0.05 mL/min.

3. The method of claim 1 or 2, wherein the first electric field is generated by applying a voltage of no greater than 30 V across the first and second electrodes and/or wherein the second electric field is generated by applying a voltage of no greater than 10 V across the first and second electrodes.

4. The method of any preceding claim, wherein the chamber has a volume of no greater than approximately 50 µL.

5. The method of any preceding claim, wherein the first electrode has a polished surface layer and/or wherein the distance between the first and second electrodes is no greater than 0.5 mm.

6. The method of any preceding claim, wherein the radioisotope is trapped on the first electrode with an efficiency of at least 94% or released from the first electrode with an efficiency of at least 96%.

7. The method of any preceding claim, wherein the method further comprises removing the aqueous solution from the chamber prior to providing the flow of the organic-based solution to the chamber and optionally the method further comprises washing the chamber after trapping the radioisotope on the first electrode and before providing the flow of an organic-based solution to the chamber.

8. The method of any preceding claim, wherein the organic-based solution comprises an organic solvent wherein optionally the organic solvent further comprises from 1% to 10% H₂O.

9. The method of any preceding claim, wherein the method further comprises heating the chamber and/or the organic based solution to a temperature of from 50 to 100 °C prior to generating the second electric field and/or wherein the method further comprises reacting the radioisotope released from the first electrode with a precursor to provide a radiopharmaceutical or an intermediate in the synthesis of a radiopharmaceutical and optionally wherein the method comprises transferring the organic-based solution containing the released radioisotope to a reactor in which the radioisotope is reacted with the precursor and further optionally wherein the organic-based solution comprises the precursor such that the radioisotope reacts with the precursor in the chamber upon release of the radioisotope from the first electrode.

10. The method of any preceding claim, wherein the chamber forms a part of a microfluidic cell.

11. An microfluidic apparatus (10) for separating and recovering a radioactive isotope from an aqueous solution comprising the radioactive isotope, the apparatus comprising:
an inlet (26a);
an outlet (26b); and
a chamber in fluid communication with the inlet and the outlet to form a fluid pathway, the chamber comprising a first electrode (18) and a second electrode (30), wherein the first electrode is formed from a carbon rod and has a flat surface comprising a plurality of recesses;
wherein the chamber has a volume capacity of no greater than about 50 µL; and
wherein the distance between the first electrode and the second electrode is no greater than 0.5 mm.

12. The apparatus of claim 11, wherein the surface area of the first electrode which comes into contact with the flow of aqueous solution is at least 20 mm².

13. The apparatus of claim 11 or claim 12, wherein the first electrode has a polished surface layer, wherein optionally the apparatus is configured to receive fluid at a flow rate of at least 0.1 mL/min.

14. The apparatus of any one of claims 11 to 13, wherein the second electrode is made of platinum and/or wherein the first electrode has a hardness of at least 2.0 on the Mohs scale, and/or wherein the chamber has a volume capacity of no greater than about 30 µL.

15. The apparatus of any one of claims 11 to 14, further comprising a heater and optionally wherein the apparatus is a microfluidic cell.

## Patentansprüche

1. Verfahren zum Trennen und Rückgewinnen eines Radioisotops aus einer wässrigen Lösung umfassend das Radioisotop, das Verfahren umfassend ein Verwenden einer mikrofluidischen Vorrichtung (10) umfassend eine Kammer:
Leiten der wässrigen Lösung zu der Kammer, wobei die Kammer eine erste Elektrode (18) und eine zweite Elektrode (30) umfasst;
Erzeugen eines ersten elektrischen Felds zwischen der ersten und der zweiten Elektrode, wodurch das Radioisotop an der ersten Elektrode eingefangen wird;
Leiten einer Lösung auf organischer Basis zu der Kammer, die die erste und die zweite Elektrode umfasst; und
Erzeugen eines zweiten elektrischen Felds zwischen der ersten und der zweiten Elektrode, wobei das zweite elektrische Feld eine dem ersten elektrischen Feld entgegengesetzte Polarität aufweist, wodurch das radioaktive Isotop von der ersten Elektrode in die Lösung auf organischer Basis freigesetzt wird, wobei die erste Elektrode aus einem Kohlenstoffstab oder einem Abschnitt davon gebildet ist und eine flache Oberfläche aufweist, die eine Vielzahl von Aussparungen umfasst.

2. Verfahren nach Anspruch 1, wobei das Verfahren ein Bereitstellen eines Durchflusses der wässrigen Lösung mit einer Flussrate von mindestens 0,1 ml/Min. umfasst, und/oder das Verfahren ein Bereitstellen eines Durchflusses der Lösung auf organischer Basis mit einer Flussrate von mindestens 0,05 ml/Min. umfasst.

3. Verfahren nach Anspruch 1 oder 2, wobei das erste elektrische Feld durch Anlegen einer Spannung von nicht mehr als 30 V über die erste und die zweite Elektrode erzeugt wird und/oder wobei das zweite elektrische Feld durch Anlegen einer Spannung von nicht mehr als 10 V über die erste und die zweite Elektrode erzeugt wird.

4. Verfahren nach einem der vorherigen Ansprüche, wobei die Kammer ein Volumen von nicht mehr als etwa 50 µl aufweist.

5. Verfahren nach einem der vorherigen Ansprüche, wobei die erste Elektrode eine polierte Oberflächenschicht aufweist und/oder wobei der Abstand zwischen der ersten und der zweiten Elektrode nicht größer als 0,5 mm ist.

6. Verfahren nach einem der vorherigen Ansprüche, wobei das Radioisotop an der ersten Elektrode mit einem Wirkungsgrad von mindestens 94 % eingefangen oder von der ersten Elektrode mit einem Wirkungsgrad von mindestens 96 % freigesetzt wird.

7. Verfahren nach einem der vorherigen Ansprüche, wobei das Verfahren ferner ein Entfernen der wässrigen Lösung aus der Kammer umfasst, bevor der Kammer der Durchfluss der Lösung auf organischer Basis zugeführt wird, und optional das Verfahren ferner ein Waschen der Kammer nach dem Einfangen des Radioisotops an der ersten Elektrode und vor dem Zuführen des Durchflusses einer Lösung auf organischer Basis zu der Kammer umfasst.

8. Verfahren nach einem der vorherigen Ansprüche, wobei die Lösung auf organischer Basis ein organisches Lösungsmittel umfasst, wobei optional das organische Lösungsmittel ferner 1% bis 10 % H₂O umfasst.

9. Verfahren nach einem der vorherigen Ansprüche, wobei das Verfahren ferner ein Erhitzen der Kammer und/oder der Lösung auf organischer Basis auf eine Temperatur von 50 bis 100 °C vor Erzeugen des zweiten elektrischen Felds umfasst, und/oder wobei das Verfahren ferner ein Reagieren des von der ersten Elektrode freigesetzten Radioisotops mit einem Vorläufer umfasst, um ein Radiopharmazeutikum oder ein Zwischenprodukt in der Synthese eines Radiopharmazeutikums bereitzustellen, und optional wobei das Verfahren ein Übertragen der Lösung auf organischer Basis, die das freigesetzte Radioisotop enthält, in einen Reaktor umfasst, in dem das Radioisotop mit dem Vorläufer reagiert wird, und ferner optional wobei die Lösung auf organischer Basis den Vorläufer umfasst, sodass das Radioisotop nach Freisetzung des Radioisotops von der ersten Elektrode in der Kammer mit dem Vorläufer reagiert.

10. Verfahren nach einem der vorherigen Ansprüche, wobei die Kammer einen Teil einer mikrofluidischen Zelle bildet.

11. Mikrofluidische Vorrichtung (10) zum Trennen und Rückgewinnen eines radioaktiven Isotops aus einer wässrigen Lösung umfassend das radioaktive Isotop, die Vorrichtung umfassend:
einen Einlass (26a);
einen Auslass (26b); und
eine Kammer in Fluidverbindung mit dem Einlass und dem Auslass, um einen Fluidweg zu bilden, die Kammer umfassend eine erste Elektrode (18) und eine zweite Elektrode (30),
wobei die erste Elektrode aus einem Kohlenstoffstab gebildet ist und eine ebene Oberfläche mit einer Vielzahl von Aussparungen aufweist;
wobei die Kammer eine Volumenkapazität von nicht mehr als etwa 50 µl aufweist; und wobei der Abstand zwischen der ersten Elektrode und der zweiten Elektrode nicht größer als 0,5 mm ist.

12. Vorrichtung nach Anspruch 11, wobei die Oberfläche der ersten Elektrode, die mit dem Durchfluss der wässrigen Lösung in Kontakt kommt, mindestens 20 mm² ist.

13. Vorrichtung nach Anspruch 11 oder Anspruch 12, wobei die erste Elektrode eine polierte Oberflächenschicht aufweist, wobei optional die Vorrichtung konfiguriert ist, um Fluid mit einer Flussrate von mindestens 0,1 ml/Min. aufzunehmen.

14. Vorrichtung nach einem der Ansprüche 11 bis 13, wobei die zweite Elektrode aus Platin besteht und/oder wobei die erste Elektrode eine Härte von mindestens 2,0 auf der Mohs-Skala aufweist und/oder wobei die Kammer eine Volumenkapazität von nicht mehr als etwa 30 µl aufweist.

15. Vorrichtung nach einem der Ansprüche 11 bis 14, ferner umfassend ein Heizgerät und optional wobei die Vorrichtung eine mikrofluidische Zelle ist.

## Revendications

1. Procédé de séparation et de récupération d'un radio-isotope à partir d'une solution aqueuse comprenant le radio-isotope, le procédé comprenant l'utilisation d'un dispositif microfluidique (10) comprenant une chambre :
l'écoulement de la solution aqueuse vers la chambre, la chambre comprend une première électrode (18) et une seconde électrode (30) ;
la génération d'un premier champ électrique entre les première et seconde électrodes, piégeant ainsi le radio-isotope sur la première électrode ;
l'écoulement d'une solution à base organique vers la chambre comprenant les première et seconde électrodes ; et
la génération d'un deuxième champ électrique entre les première et seconde électrodes, dans lequel le deuxième champ électrique a une polarité opposée au premier champ électrique, libérant ainsi l'isotope radioactif de la première électrode dans la solution à base organique, dans lequel la première électrode est formée à partir d'une tige de carbone ou d'une section de celle-ci et a une surface plane comprenant une pluralité d'évidements.

2. Procédé selon la revendication 1, dans lequel le procédé comprend la fourniture d'un écoulement de la solution aqueuse à un débit d'au moins 0,1 ml/min et / ou le procédé comprend la fourniture d'un écoulement de la solution à base organique à un débit d'au moins 0,05 ml/min.

3. Procédé selon la revendication 1 ou 2, dans lequel le premier champ électrique est généré en appliquant une tension non supérieure à 30 V à travers les première et seconde électrodes et / ou dans lequel le second champ électrique est généré en appliquant une tension non supérieure à 10 V à travers les première et seconde électrodes.

4. Procédé selon l'une quelconque des revendications précédentes, dans lequel la chambre a un volume non supérieur à environ 50 µl.

5. Procédé selon l'une quelconque des revendications précédentes, dans lequel la première électrode a une couche de surface polie et / ou dans lequel la distance entre les première et seconde électrodes n'est pas supérieure à 0,5 mm.

6. Procédé selon l'une quelconque des revendications précédentes, dans lequel le radio-isotope est piégé sur la première électrode avec une efficacité d'au moins 94 % ou libéré de la première électrode avec une efficacité d'au moins 96 %.

7. Procédé selon l'une quelconque des revendications précédentes, dans lequel le procédé comprend en outre l'élimination de la solution aqueuse de la chambre avant de fournir l'écoulement de la solution à base organique vers la chambre et facultativement le procédé comprend en outre le lavage de la chambre après avoir piégé le radio-isotope sur la première électrode et avant de fournir l'écoulement d'une solution à base organique à la chambre.

8. Procédé selon l'une quelconque des revendications précédentes, dans lequel la solution à base organique comprend un solvant organique dans lequel facultativement le solvant organique comprend en outre de 1% à 10% de H₂O.

9. Procédé selon l'une quelconque des revendications précédentes, dans lequel le procédé comprend en outre le chauffage de la chambre et / ou de la solution à base organique à une température de 50 à 100° C avant de générer le deuxième champ électrique et / ou dans lequel le procédé comprend en outre la réaction du radio-isotope libéré de la première électrode avec un précurseur pour fournir un produit radiopharmaceutique ou un intermédiaire dans la synthèse d'un produit radiopharmaceutique et facultativement dans lequel le procédé comprend le transfert de la solution à base organique contenant le radio-isotope libéré dans un réacteur dans lequel le radio-isotope est mis à réagir avec le précurseur et en outre facultativement dans lequel la solution à base organique comprend le précurseur de telle sorte que le radio-isotope réagisse avec le précurseur dans la chambre lors de la libération du radio-isotope de la première électrode.

10. Procédé selon l'une quelconque des revendications précédentes, dans lequel la chambre fait partie d'une cellule microfluidique.

11. Appareil microfluidique (10) de séparation et récupération d'un isotope radioactif à partir d'une solution aqueuse comprenant l'isotope radioactif, l'appareil comprenant :
une entrée (26a) ;
une sortie (26b) ; et
une chambre en communication fluidique avec l'entrée et la sortie pour former un chemin de fluide, la chambre comprenant une première électrode (18) et une seconde électrode (30),
dans lequel la première électrode est formée d'une tige de carbone et a une surface plane comprenant une pluralité d'évidements ;
dans lequel la chambre a une capacité volumique non supérieure à environ 50 µl ; et dans lequel la distance entre la première électrode et la seconde électrode n'est pas supérieure à 0,5 mm.

12. Appareil selon la revendication 11, dans lequel la surface de la première électrode qui entre en contact avec l'écoulement de solution aqueuse est d'au moins 20 mm².

13. Appareil selon la revendication 11 ou la revendication 12, dans lequel la première électrode a une couche de surface polie, dans lequel facultativement l'appareil est configuré pour recevoir un fluide à un débit d'au moins 0,1 ml/min.

14. Appareil selon l'une quelconque des revendications 11 à 13, dans lequel la seconde électrode est en platine et / ou dans lequel la première électrode a une dureté d'au moins 2,0 sur l'échelle de Mohs, et / ou dans lequel la chambre a une capacité volumique non supérieure à environ 30 µl.

15. Appareil selon l'une quelconque des revendications 11 à 14, comprenant en outre un élément chauffant et facultativement dans lequel l'appareil est une cellule microfluidique.
